# EUROPÄISCHE PATENTSCHRIFT

(11) **EP 2 830 518 B1**
(45) Veröffentlichungstag und Bekanntmachung des Hinweises auf die Patenterteilung: **19.06.2019**
(21) Anmeldenummer: 12711790.1
(22) Anmeldetag: 28.03.2012
(51) Int. Cl.: A61B 17/70, A61B 17/80, A61B 17/86

(54) **VERSTÄRKUNGS-IMPLANTAT FÜR LAMINA MIT EINEM FREITRAGENDEN BRÜCKENTEIL**
REINFORCEMENT IMPLANT FOR LAMINA WITH A CANTILEVER BRIDGE PART
IMPLANT DE RENFORCEMENT D'UNE LAME VERTÉBRALE, MUNI D'UNE PARTIE PONT EN PORTE-À-FAUX

(43) Veröffentlichungstag der Anmeldung: 04.02.2015
(73) Patentinhaber: FACET-LINK Inc., Rockaway NJ 07866 (US)
(72) Erfinder: JENSEN, Harm-Iven, 24214 Noer (DE); LINK, Helmut, D., 22397 Hamburg (DE)
(74) Vertreter: Glawe, Delfs, Moll
(86) Internationale Anmeldenummer: PCT/EP2012/001357
(87) Internationale Veröffentlichungsnummer: WO 2013/143558

(56) Entgegenhaltungen:
- WO-A1-2005/096969
- WO-A1-2011/040983
- US-A1- 2007 185 489
- US-A1- 2011 106 083
- US-A1- 2011 172 666
- US-A1- 2012 022 603
- US-A1- 2012 071 923

## Beschreibung

Die Erfindung betrifft ein Verstärkungs-Implantat für Lamina mit einem freitragenden Brückenteil (im weiteren Verlauf auch als Freitrageteil genannt).

Wirbelsäulen von Menschen oder Tieren sind aufgebaut aus einer Mehrzahl von übereinander angeordneten Wirbeln. Sie sind sowohl lasttragend wie auch gelenkig miteinander verbunden. Dazu weisen die Wirbel einen Aufbau auf mit einem massiven Wirbelkörper mit zwei seitlich nach hinten vorstehenden knöchernen Vorsprüngen (Pedikel), die in ihrem hinteren Teil über einen knöchernen Bogen verbunden sind. Im Verbindungsbereich ist der Knochenbogen verbreitert (Lamina) und weist in seiner Mitte einen nach hinten abragenden spinalen Vorsprung (Dorn) auf. Der Dorn wie auch zwei weitere transversale Vorsprünge an den Seitenflächen der Pedikel bilden Anlenkpunkte für Muskeln und Bänder. In dem Bereich, wo die Pedikel in die Lamina übergehen, sind auf jeder Seite ein oberer und ein unterer Gelenkvorsprung angeordnet. Sie bilden jeweils Teil eines Facettengelenks mit einem benachbarten oberen beziehungsweise unteren Wirbel. Zur lasttragenden Verbindung mit dem benachbarten oberen und unteren Wirbel ist jeweils eine Bandscheibe vorgesehen, die unten beziehungsweise oben auf verhältnismäßig ebenen Deckflächen des Wirbelkörpers angeordnet sind. Der von der Rückseite des Wirbelkörpers und dem Wirbelbogen umgrenzte Raum bildet einen Hohlraum (Spinalkanal), in welchem parallel zur Wirbelsäule laufende Nervenstränge aufgenommen sind. Es hat sich gezeigt, dass Einklemmungen oder Einschnürungen, insbesondere aufgrund von knöchernen Zuwachsungen im Bereich des Spinalkanals oder aufgrund von Auswölbungen der Bandscheibe (sog. Bandscheibenvorfall), Druck auf die Nervenstränge ausüben, wodurch gravierende Rückenschmerzen entstehen können.

Zur Therapie ist es bekannt, den Wirbelbogen zumindest teilweise zu öffnen, um so einen Zugang zu dem Spinalkanal zu schaffen. Dort werden mittels an sich bekannter Instrumente die störenden Zuwachsungen beseitigt und so der Druck von den Nervensträngen genommen. Der durch den Druck induzierte Schmerz wird dadurch entsprechend verringert. Bei dieser auch als Laminektomie beziehungsweise Dekompression bekannten Methode wird der in der Lamina geschaffene Zugang, also die dort vorhandene Öffnung, nach der Operation meist nicht verschlossen. Es hat sich gezeigt, dass hierdurch die mechanische Stabilität des Wirbels geschwächt ist.

Es ist von der Anmelderin in einer früheren Patentanmeldung vorgeschlagen worden, einen Implantat-Satz mit Verstärkungsimplantaten in verschiedenen Größen bereitzustellen. Sie weisen einen rhombusförmigen Füllkörper auf zum Einsetzen in und Auffüllen der durch die Laminektomie geschaffenen Öffnung. Der rhombusförmige Füllkörper liegt mit seinen beiden gegenüberliegenden Lateralflächen an den Schnittflächen der Lamina an. Damit ist der Laminabogen durch das Einsetzen des Füllkörpers wieder komplett, so dass er wieder Last tragen kann und insbesondere unter Drucklasten nicht kollabiert. Um den resezierten Bereich möglichst vollständig und ohne Aufspreizen füllen zu können, muss das Verstärkungsimplantat in einer beträchtlichen Anzahl (mindestens sieben) verschiedener Größen je Seite (links oder rechts) vorgesehen werden. Das bedingt eine beträchtliche Komplexität des Implantat-Satzes. Außerdem ist es für die angestrebte Druckübertragungsfunktion wichtig, dass der Füllkörper mit seinen Lateralseiten möglichst flächig an den Schnittflächen der Lamina anliegt. Da in der Praxis die Schnittflächen häufig nicht ganz eben sind, ist die Druckübertragung verschlechtert. Dazu kommt, dass das Einsetzen des Füllkörpers bei nicht ebenen Schnittflächen erschwert ist, was zusätzliche Komplikationen hervorruft.

Ein Verstärkungs-Implantat mit den Merkmalen des Oberbegriffs der Ansprüche 1 und 15 ist aus der US 2012/071923 A1 bekannt.

Der Erfindung liegt die Aufgabe zugrunde, ein verbessertes Verstärkungsimplantat zu schaffen, dass diese Nachteile vermeidet.

Die erfindungsgemäße Lösung liegt in den Merkmalen der unabhängigen Ansprüche. Vorteilhafte Weiterbildungen sind Gegenstand der abhängigen Ansprüche.

Bei einem Verstärkungsimplantat zum Einsetzen in die Lamina eines Wirbels, das einen Grundkörper mit Anlageflächen an den Wirbel und eine Befestigungseinrichtung umfasst, sind erfindungsgemäß vorgesehen ein Freitrageteil zur Überspannung eines resezierten Teils und an gegenüberliegenden Enden des Freitrageteils je ein Verankerungsteil, wobei ein erster Verankerungsteil mit einer Druckfläche zur Anlage am Dornfortsatz des Wirbels und ein zweiter Verankerungsteil mit einer Querschubfläche zur Auflage auf einer Außenseite der Lamina ausgebildet sind, und die Druckfläche und die Querschubfläche einen stumpfen Winkel einschließen, wobei an der Querschubfläche eine Scherstoppeinrichtung insbesondere eine Facettenschraube, angeordnet ist, und an einem Rand der Querschubfläche zur Überspannung des resezierten Teils der Lamina ein lasttragender Bereich des Freitrageteils anschließt.

Die Erfindung beruht auf dem Gedanken, mittels der speziellen Verankerungsteile eine haltbare und im Betrieb robuste Überbrückung des resezierten Laminasegments zu schaffen, die zudem noch leicht implantierbar ist. Mit den beiden stumpfwinklig zueinander orientierten Anlageflächen, der Druckfläche einerseits und der Querschubfläche andererseits, wird eine in allen Raumdimensionen sichere und zwängungsfreie Halterung geschaffen. Mit dieser Konstruktion wird eine statische Überbestimmung vermieden, wie sie für Implantate mit zwei im wesentlichen parallel gegenüberliegenden Druckflächen (insbesondere als Füllkörper ausgeführte) typisch ist. Die natürliche Elastizität im Knochen wird auf diese Weise aufgenommen und bleibt so erhalten, anstatt sie durch Verzwängung zu beschränken. Das Implantat verhält sich damit physiologischer. Das ist nicht nur günstiger für das Verhalten, sondern bedeutet auch eine erhöhte Dauerhaltbarkeit des Implantats durch Vermeidung von Degeneration. Denn es hat sich gezeigt, dass sehr steife Implantate, wie es verzwängende Implantate sind, leicht zu einer Degeneration des nun entlasteten Knochens führen.

Zudem ist das erfindungsgemäße Implantat in der Handhabung bei der Implantation selbst vereinfacht. Denn es braucht nicht in den durch die Resektion an der Lamina geschaffen Freiraum eingeführt zu werden, sondern wird vielmehr von außen sozusagen aufgesetzt, um so den Freiraum zu überbrücken. Dazu weist das Implantat an seiner einen Seite eine Druckfläche auf, welche gegen eine Seitenfläche des Dornfortsatzes am Wirbel gesetzt wird, und das Implantat weist auf seiner anderen Seite eine Querschubfläche auf, die auf die Außenseite der Lamina platziert wird und dort mit einer Scherstoppeinrichtung fixiert wird. Das Implantat braucht daher gar nicht in den Freiraum hineingeschoben zu werden. Selbst mit den eigentlichen Resektionsflächen, die durch die Resektion in der Lamina geschaffen wurden, hat es keinen lasttragenden Kontakt. Unebenheiten der Resektionsfläche, wie sie in der operativen Praxis häufig unvermeidlich sind, sind damit ohne Einfluss auf die Lage und die Befestigung des Implantats.

Vorzugsweise ist das Freitrageteil des Verstärkungsimplantats so ausgebildet, dass es mit seinem die Lastkräfte von der Querschubfläche zur Druckfläche übertragenen Bereich überschneidungsfrei ist in Bezug auf eine durch die Querschubfläche definierte Ebene. Das bedeutet, dass der lasttragende Bereich des Freitrageteils nicht in den durch die Resektion an der Lamina geschaffenen Freiraum hineinragt; der Brückenteil befindet sich also komplett außerhalb. Irritationen durch die Kraftübertragung von der Querschubfläche auf die Druckfläche auf den besonders empfindlichen resezierten Bereich der Lamina können damit weitestgehend verhindert werden.

Zweckmäßigerweise ist das Verstärkungsimplantat so ausgebildet, dass die Verankerungsteile als erster und zweiter Schenkel ausgeführt sind, die über den Freitrageteil verbunden sind. Mit dieser Schenkelbauweise wird eine material- und raumsparende Gestaltung des Implantats ermöglicht. Durch die raumsparende Gestaltung wird die Einwirkung auf umliegendes Gewebe und damit die Gefahr von durch das Implantat hervorgerufenen Irritationen minimiert. An mindestens einem der Schenkel ist ein Schwenkgelenk für einen Befestigungsstift angeordnet. Unter einem Befestigungsstift wird insbesondere eine Schraube oder ein Knochennagel verstanden. Mittels dieses Schwenkgelenks kann die Achse des Befestigungsstifts in gewissen Grenzen frei eingestellt werden. Bewährt hat sich eine Verstellbarkeit um 15° in jede Richtung bezogen auf eine Mittellage ("Normallage") .

Vorzugsweise weist das Schwenkgelenk einen kalottenförmigen Aufnahmesitz und einen darin gelagerten Ring auf, durch den der Befestigungsstift geführt ist. Durch die kalottenförmige Gestaltung ergibt sich eine stufenlose Verschwenkbarkeit, die im entspannten Zustand reibungsarm und im gespannten Zustand des Befestigungsstifts selbsthemmend wird.

Besonders bevorzugt ist es, wenn der Ring eine Rotationssperre aufweist, welche ihn drehfest gegenüber dem Aufnahmesitz des Schwenkgelenks hält. Mit einer solchen Rotationssperre wird ein unerwünschtes Verdrehen des Rings in dem Schwenkgelenk verhindert. Eine solche unerwünschte Verdrehung kann herkömmlicherweise dann auftreten, wenn der Befestigungsstift eine Schraube ist und die Schraube angezogen werden soll. Ein Mitdrehen des Rings ist hierbei ungünstig. Mit der Rotationssperre wird der Ring an einem Verdrehen um die Achse des Befestigungsstifts herum gehindert, wobei die Schwenkbarkeit des Rings jedoch nicht eingeschränkt ist.

Die Schwenkgelenke sind zweckmäßigerweise so ausgebildet, dass die Befestigungsstifte um mindestens 10° und maximal 20° in jeder Richtung um die Normallage beweglich sind. Es hat sich gezeigt, dass ein größerer Winkel im Verstellbereich zu einer Schwächung der Befestigungssicherheit und der Positionsgenauigkeit führen kann. Ein kleinerer Verstellbereich hingegen genügt den Anforderungen hinsichtlich ausreichender Universalität des erfindungsgemäßen Verstärkungsimplantats häufig nicht.

Vorzugsweise sind die Schwenkgelenke in den beiden Schenkeln so ausgebildet, dass die Befestigungsstifte der beiden Schenkel in Normallage in einer Ebene liegen. Damit wird eine Befestigungsebene aufgespannt, welche identisch für beide Schenkel gilt. Eine statische Überbestimmung, wie sie bei einer windschiefen Anordnung der Befestigungsstifte au-βerhalb einer gemeinsamen Ebene vorliegen würde, könnte hingegen zu Verzwängungen führen. Dies wird durch die Anordnung in einer gemeinsamen Ebene wirkungsvoll verhindert.

Die Scherstoppeinrichtung ist mit einer Schraube ausgeführt, welche so orientiert ist, dass sie in ihrer normalen Lage um höchstens 30° von einer Lotrechten der Querschubfläche abweicht, aber um mindestens 10°. Es hat sich gezeigt, dass mit einer solchen Anordnung zwei Ziele miteinander vereint werden können. Das eine Ziel besteht in einer hinreichenden Sicherung des erfindungsgemäßen Verstärkungs-Implantats gegenüber einer unerwünschten Verschiebung relativ zur Lamina. Das andere Ziel besteht darin, die Schraube so auszurichten, dass sie in einen mechanisch robusten Teil des Knochens zur Befestigung zielt, in dessen Verlängerung sich das Facettengelenk zu dem caudal (d.h. an der Wirbelsäule nach unten hin) anschließenden Wirbel befindet. Damit kann durch Einsetzen einer langen Schraube, einer sogenannten Facettenschraube, die bis in den benachbarten unteren Wirbel reicht, nicht nur eine Befestigung erreicht werden, sondern zugleich auch das Facettengelenk fusioniert werden. Damit wird das Facettengelenk auf dieser Seite immobilisiert. Ist eine Immobilisierung nicht vorgesehen, so genügt eine kurze Schraube, die nicht bis in den benachbarten unteren Wirbel reicht.

An dem Freitrageteil des Verstärkungsimplantats kann ein Flügelfortsatz vorgesehen sein, der von einem Rand der Querschubfläche abragt. Zweckmäßiger Weise ist der Flügelfortsatz parallel zur Druckfläche ausgerichtet. Der Flügelfortsatz ist selbst nicht lasttragend und ragt in den durch die Resektion geschaffenen Freiraum in der Lamina hinein. Er erleichtert ein Einführen des Implantats unter schwierigen Bedingungen. Weiter verhindert er, je nach Größe des Flügelfortsatzes, ein Eindringen von Knochenresten oder anderem unerwünschten Material von außen in den Spinalkanal des Wirbels. Zu diesem Zweck ist der Flügelfortsatz vorzugsweise in verschiedenen Größen vorgesehen.

Der Flügelfortsatz ist zweckmäßigerweise so ausgebildet, dass er eine plane, von der Druckfläche weg weisende Außenseite und vorzugsweise eine Verstärkungsrippe an seiner zur Druckfläche weisenden Innenseite aufweist. Die Außenseite ist dazu ausgebildet, im Bereich der lateralen Resektionsfläche der Lamina anzuliegen, wobei eine kraftschlüssige Anlage an der Resektionsfläche der Lamina nicht erforderlich ist. Je geringer der dazwischen befindliche Spalt, desto besser die Schutzwirkung gegenüber einfallendem Material. Zweckmäßigerweise ist der Flügelfortsatz einstückig mit dem Freitrageteil ausgeführt. Zur weiteren mechanischen Versteifung ist die Verstärkungsrippe an der Innenseite vorgesehen. Im implantierten Zustand befindet sich diese in dem durch die Resektion geschaffenen Freiraum und kommt nicht in Kontakt mit der Lamina.

Vorzugsweise ist der Flügelfortsatz im Übergangsbereich von der Querschubfläche zum freitragenden Teil angeordnet, und zwar zweckmäßigerweise derart, dass sich der Flügelfortsatz über maximal die halbe Weite der Querschubfläche erstreckt. Damit wird eine maximale Abdeckwirkung durch den Flügelfortsatz erreicht, und zwar ohne dass die Gefahr eines unerwünscht weiten Eindringens in den resezierten Raum bzw. den von der Lamina umschlossenen Spinalkanal mit den darin verlaufenden Nervensträngen besteht. Der Flügelfortsatz ist vorzugsweise so ausgerichtet, dass sein Unterrand divergiert in Bezug auf eine Achse der Scherstoppeinrichtung. Damit ist gemeint, dass sich der untere Rand umso mehr nach unten weg bewegt, je weiter er von der Querschubfläche entfernt ist. Mit einer solchen nach unten abragenden Gestaltung des Flügelfortsatzes wird eine optimale Abdeckung erzielt.

Es sei angemerkt, dass der Flügelfortsatz dank seiner flächigen Gestaltung auf der Außenseite und der vorzugsweise vorgesehenen Verstärkungsrippe auf der Innenseite eine Nottragefunktion aufweisen kann. Sollte sich die Befestigung über das Brückenteil lösen, beispielsweise durch Versagen der Scherstoppeinrichtung, so kann sich die Lamina mit ihrer Schnittfläche nur soweit bewegen, bis sie auf der planen Außenseite des Flügelfortsatzes anliegt und wird dort getragen. Ein Kollabieren des Wirbelbogens mit dem damit einhergehenden dramatischen Folgen für den Patienten wird auf diese Weise sicher vermieden.

Die Erfindung umfasst ferner einen Implantatsatz zum Einsetzen in die Lamina eines Wirbels, umfassend eine Mehrzahl von Verstärkungsimplantaten in verschiedenen Größen, die je einen Grundkörper mit Anlageflächen an den Wirbel und eine Befestigungseinrichtung umfassen, wobei erfindungsgemäß vorgesehen sind ein Freitrageteil zur Überspannung eines resezierten Teils und an gegenüberliegenden Enden des Freitrageteils je ein Verankerungsteil, wobei ein erster Verankerungsteil mit einer Druckfläche zur Anlage am Dornfortsatz des Wirbels und ein zweiter Verankerungsteil mit einer Querschubfläche zur Auflage auf einer Außenseite der Lamina ausgebildet sind, und die Druckfläche und die Querschubfläche einen stumpfen Winkel einschließen, wobei an der Querschubfläche eine Scherstopeinrichtung, insbesondere eine Facettenschraube, angeordnet ist, und an einem Rand der Querschubfläche zur Überspannung des resezierten Teils der Lamina ein lasttragender Bereich des Freitrageteils anschließt.

Zur näheren Erläuterung und zur weiteren optionalen Ausgestaltung wird auf die vorstehende Erklärung zu dem einzelnen Verstärkungsimplantat verwiesen.

Die Erfindung wird nachfolgend unter Bezugnahme auf die beigefügte Zeichnung anhand eines Ausführungsbeispiels näher erläutert. Es zeigen:
- Fig. 1: eine Ansicht von unten eines Ausführungsbeispiels des erfindungsgemäßen Verstärkungsimplantats;
- Fig. 2a,) b): eine Aufsicht auf das Verstärkungsimplantat bzw. eine Ansicht von lateral mit eingesetzten Facettenschrauben;
- Fig. 3:: eine Übersichtsdarstellung verschiedener Größen des Verstärkungsimplantat und zweier Varianten;
- Fig. 4a), b): eine Ansicht von lateral bzw. einer Aufsicht von oben einer zweiten Ausführungsform des Verstärkungsimplantats;
- Fig. 5a), b): eine Ansicht von lateral bzw. eine Aufsicht von oben einer dritten Ausführungsform des Verstärkungsimplantats; und
- Fig. 6a)-c): einen Wirbel mit einer Laminaresektion mit und ohne eingesetztes Verstärkungsimplantat gemäß der zweiten Ausführungsform nach Fig. 4.

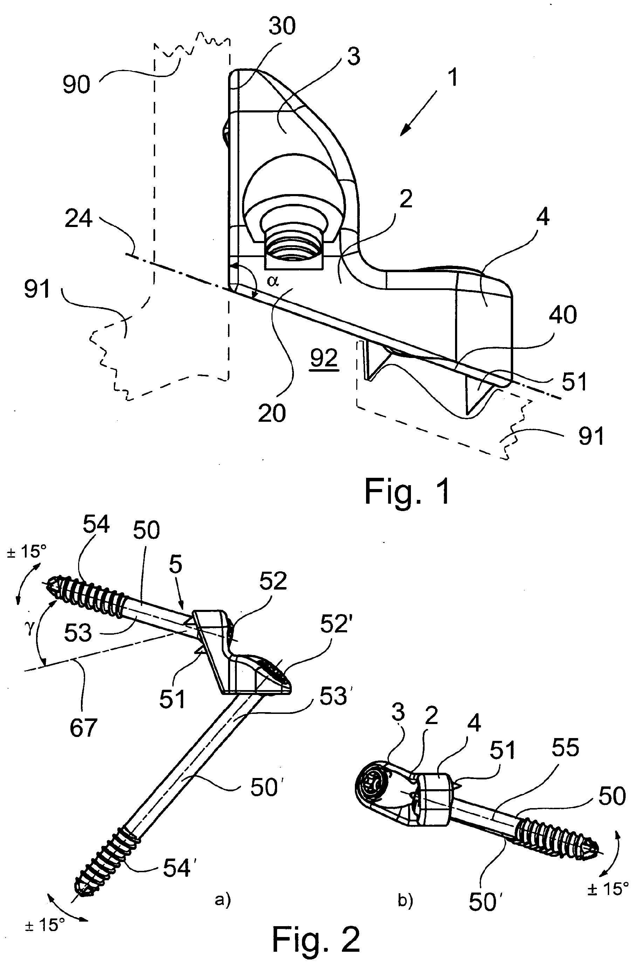

In Figur 1 ist ein erstes Ausführungsbeispiel für ein Verstärkungsimplantat gemäß der Erfindung dargestellt. Es ist in seiner Gesamtheit mit der Bezugsziffer 1 bezeichnet. Es ist im wesentlichen schenkelförmig ausgebildet mit einem ersten Schenkel 3 und einem zweiten Schenkel 4, die über ein Brückenteil 2 miteinander verbunden sind.

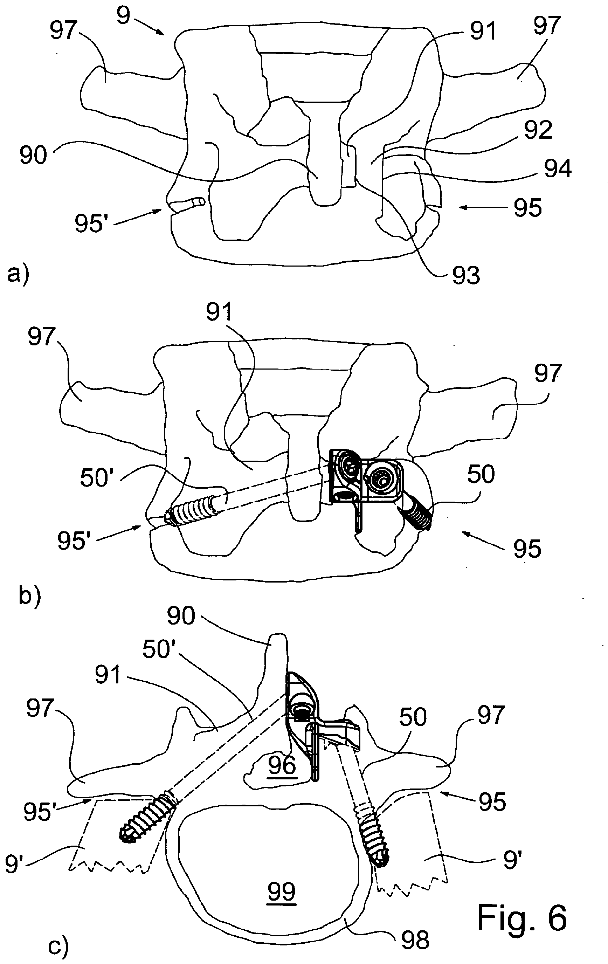

Zum besseren Verständnis der Erfindung seien nachfolgend der Aufbau des Wirbels und die Art der Interaktion zwischen dem Verstärkungsimplantat und dem Wirbel näher erläutert. Es wird insbesondere Bezug genommen auf Figur 6a) bis c). Der Wirbel 9 weist einen massiven Wirbelkörper 98 mit zwei seitlich vorragenden knöchernen Vorsprüngen 97 auf, die in ihrem hinteren Bereich über einen knöchernen Bogen verbunden sind. Der knöcherne Bogen umfasst eine Lamina 91 und in deren Mitte einen nach hinten abragenden Vorsprung (Dornfortsatz) 90. In dem Bereich des Übergangs in die Lamina 91 sind auf jeder Seite ein oberer und ein unterer Gelenkvorsprung angeordnet, die jeweils Teil eines Facettengelenks 95, 95' zu einem benachbarten unteren Wirbel 9' bilden. Der Wirbel 9 ist mit seinem benachbarten unteren Wirbel ferner über eine Bandscheibe 99 verbunden, die lasttragend zwischen einer unteren Deckfläche des Wirbelkörper 98 der entsprechenden oberen Deckfläche des unteren benachbarten Wirbels 9' angeordnet ist. In Figur 6a) erkennt man in der Rückansicht, dass im Bereich der Lamina 91 rechtsseitig von dem Dornfortsatz 90 ein Freiraum 92 besteht. Dieser wurde geschaffen durch eine Resektion, wodurch links und rechts des Freiraums 92 entsprechenden Schnittflächen (Resektionsflächen) 93, 94 an der Lamina 91 entstanden sind. Die durch diesen Freiraum 92 geschaffene Öffnung bildet einen Zugang zu einem Spinalkanal 96. Sie wird mit dem erfindungsgemäßen Verstärkungsimplantat 1 verschlossen und mechanisch stabilisiert.

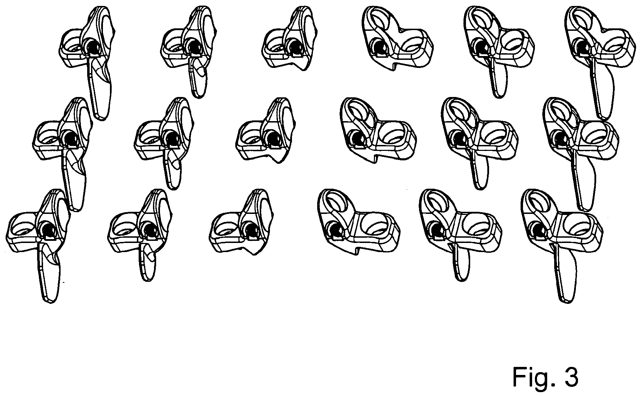

Wie in Fig. 6a und 6b dargestellt ist, wird das erfindungsgemäße Verstärkungsimplantat von hinten, also von posterior, auf die Lamina 91 aufgesetzt, und zwar in der Art, dass es mit seinem ersten Schenkel 3 am Dornfortsatz 90 anlegt und mit seinem zweiten Schenkel 4 auf der Rückseite des Bereichs der Lamina 91 unmittelbar rechtsseitig der Resektionsfläche 94 aufliegt. Dargestellt in Fig. 6a) bis c) ist eine rechtsseitige Implantation; genauso gut kann eine linksseitige Implantation vorgesehen sein, mit einem entsprechend spiegelbildlich gestalteten Verstärkungsimplantat (vergleiche Fig. 3).

Zur Befestigung des Verstärkungsimplantats 1 an dem Wirbel 9 ist an dem ersten Schenkel 3 an seiner Außenseite eine Druckfläche 30 angeordnet. Die Druckfläche 30 ist im wesentlichen plan ausgebildet. An dem zweiten Schenkel 4 ist an seiner Außenseite eine Querschubfläche 40 angeordnet, die zur Auflage auf der Außenseite einer Lamina 91 eines Wirbels 9 ausgebildet ist. Für die Querschubfläche 40 ist eine Scherstoppeinrichtung 5 vorgesehen. Sie umfasst in dem dargestellten Ausführungsbeispiel Dorne 51 (dargestellt sind zwei, jedoch kann auch eine andere kleinere oder größere Anzahl vorgesehen sein) und eine Facettenschraube 50 (siehe Figur 2). Die Facettenschraube 50 ist so ausgerichtet, dass sie in ihrer Normallage mit ihrer Achse 55 einen Winkel γ von 30° zur Lotrechten 67 der Querschubfläche 40 bildet.

Die Facettenschraube 50 ist mit einem Kopf 52, einem gewindefreien Schaft 53 und einem Knochengewinde 54 an ihrem äußeren Ende versehen. Die Länge des gewindefreien Schafts 53 ist so bemessen, dass die Facettenschraube 50 mit ihm vollständig in einem diesseitigen Teil des Facettengelenks 95 zu liegen kommt, während der Teil des Schafts mit dem Knochengewinde 54 ausschließlich in einem jenseitigen Teil des Facettengelenks an den benachbarten unteren Wirbel 9' zu liegen kommt. Damit wird erreicht, dass beim Festziehen der Schraube 50 der jenseitige Teil des Facettengelenks 95 unter der Krafteinwirkung des Knochengewindes 54 zum Kopf 52 der Schraube hin gezogen wird, und somit gegen den diesseitigen Teil des Facettengelenks 95 verspannt wird. Damit wird eine sichere Immobilisierung des Facettengelenks 95 erreicht.

Es ist eine zweite Facettenschraube 50' vorgesehen, welche in den ersten Schenkel 3 eingesetzt ist. Diese Facettenschraube 50' ist so ausgerichtet, dass sie auf das auf der anderen Seite des Wirbels gelegenen Facettengelenk 95' ausgerichtet ist. Der Aufbau der zweiten Facettenschraube 50' entspricht vom Grundsatz her demjenigen der Facettenschraube 50. Sie umfasst einen Kopf 52', einen gewindefreien Schaft 53' sowie ein Knochengewinde 54'. Die Länge des gewindefreien Schafts 53' ist bedeutend größer bezogen auf den Schaft 53, da die Strecke bis zum auf der anderen Seite liegenden Facettengelenk 95' bedeutend größer ist. Diese zweite Facettenschraube 50' wird auch als translaminare Schraube 50' bezeichnet.

Ist lediglich eine Fixierung des Verstärkungsimplantats 1 gewünscht ohne Immobilisierung des Facettengelenks 95, 95', so sind die Schrauben 50, 50' um soviel kürzer, dass sie vollständig im Wirbel 9 aufgenommen sind, also nicht in den jenseitigen Teil des Facettengelenks am benachbarten unteren Wirbel 9' ragen ("kurze Schraube"). Es kann auch eine Sonderschraube vorgesehen sind, die ein Gewinde über die volle Länge des Schafts aufweist.

Die Facettenschrauben 50, 50' sind nicht starr in dem ersten bzw. zweiten Schenkel 3, 4 gelagert, sondern sind bezüglich ihrer Schraubenachse schwenkbeweglich gelagert, und zwar um einen Winkel von 15° in jeder Richtung. Dazu ist für jede Facettenschraube 50, 50' sowohl in dem Schenkel 3, wie auch in dem Schenkel 4 jeweils ein Schwenkgelenk 6 vorgesehen. Das Schwenkgelenk 6 umfasst einen kalottenförmigen Lagersitz 60, in welchem ein mit einer kugelförmigen Mantelfläche versehener Ring 61 eingesetzt ist.

Die beiden Schenkel 3, 4 sind so geformt, dass sie mit ihren Außenseiten und den daran angeordneten Druckfläche 30 und Querschubfläche 40 einen stumpfen Winkel α einschließen. Der Winkel α beträgt vorzugsweise zwischen 95° und 125°, in dem dargestellten Ausführungsbeispiel beträgt er 110°. Dank dieses stumpfen Winkels kann das Verstärkungsimplantat von dorsal implantiert werden, so dass es den durch die Resektion an der Lamina 91 geschaffenen Freiraum 92 überbrückt. Dazu liegt das Verstärkungsimplantat 1 mit seinem zweiten Schenkel 4 und der daran angeordneten Querschubfläche 40 auf der posterioren Seite der Lamina 91 auf. Dies bildet den einen Verankerungsteil. Der andere Verankerungsteil ist gebildet durch den ersten Schenkel 3 mit der daran angeordneten Druckfläche 30, welche gegen eine Seitenfläche des Dornfortsatzes 90 des Wirbels 9 gepresst ist. Das zwischen den beiden Schenkeln 3, 4 befindliche Freitrageteil 2 überspannt damit brückenartig den durch die Resektion geschaffenen Freiraum 92. Die Kraftübertragungslinien zwischen den beiden Schenkeln 3, 4 laufen durch einen lasttragenden Bereich 20 des Freitrageteils 2, und zwar in der Weise, dass der Lastfluss vollständig außerhalb des Freiraums 92 stattfindet der. Strukturell bedeutet dies, dass die Kraftübertragungslinien in dem lasttragenden Bereich 20 derart laufen, dass sie die durch die Querschubfläche 40 definierte Ebene 24 nicht schneiden, sondern ausschließlich außerhalb (d.h. posterior) davon verlaufen.

Um den zweiten Schenkel 4 mit seiner Querschubfläche 40 sicher an der Lamina 91 zu verankern, und insbesondere eine unerwünschte Scherbewegung gegenüber der Lamina 91 zu verhindern, sind als Scherstoppeinrichtung 5 sowohl die Dorne 51 wie auch die Facettenschraube 50 als Befestigungsstift vorgesehen. An sich ist jede der beiden genannten Einrichtungen für sich bereits ausreichend, um eine unerwünschte Scherbewegung zu stoppen. Zur Erhöhung der Befestigungssicherheit und um ein Abheben der Querschubfläche 40 von der Außenseite der Lamina 91 zu verhindern, ist die Facettenschraube 50 vorgesehen. Zur Verhinderung der unerwünschten Scherbewegung ist es an sich nicht erforderlich, dass die Schraube 50 die in Figur 2 dargestellte Länge aufweist. Es genügt auch eine deutlich kürzere Schraube 50, die so kurz ist, dass sie vollständig innerhalb des Wirbels 9 verbleibt. Lediglich in den Fällen, wenn mit der Schraube 50 zusätzlich die Funktionalität einer Immobilisierung des Facettengelenks 95 erreicht werden soll, ist die Schraube 50 bezüglich ihrer Länge so dimensioniert, dass sie mit ihrem Gewinde 54 aus dem Wirbel 9 herausragt und in den Facettengelenkteil des unteren, benachbarten Wirbelkörpers 9' eindringt, um das Facettengelenk 95 somit zu immobilisieren.

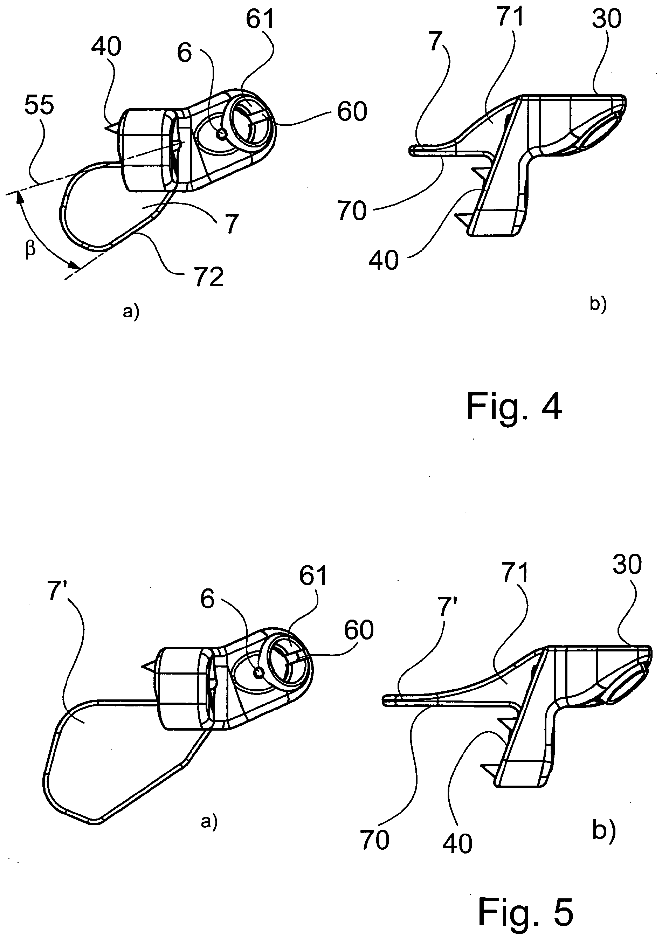

Bei einer zweiten und dritten Ausführungsform des erfindungsgemäßen Verstärkungsimplantats, wie es in den Figuren 4 und 5 dargestellt ist, ist zusätzlich ein Flügelfortsatz 7 vorgesehen. Es wird im Folgenden Bezug genommen auf die Figuren 4a) und b). Der Flügelfortsatz 7 ragt von der Querschubfläche 40 ab. Genauer gesagt ist er angeordnet im unteren Drittel der Querschubfläche 40 im Bereich des Übergangs zwischen dem zweiten Schenkel 4 und dem Freitrageteil 2, also in einem Übergang zwischen Querschubfläche 40 und Freitrageteil 20. Der Flügelfortsatz 7 ist so orientiert, dass er parallel zur Druckfläche 30 an dem ersten Schenkel 3 ausgerichtet ist der. Der Flügelfortsatz 7 weist an seiner zu der Querschubfläche 40 weisenden Außenseite 70 eine plane Fläche auf. An seiner gegenüberliegenden, zur Druckfläche 30 hin orientierten Innenseite ist er mit einer Verstärkungsrippe 71 versehen. Der Flügelfortsatz 7 umfasst mit seinem unteren Bereich den zweiten Schenkel 4, so dass er insgesamt nach schräg unten (bezogen auf den implantierten Zustand des Verstärkungsimplantat 1') abragt. Seine Unterkante 72 ist so orientiert, dass sie nach außen hin divergiert in Bezug auf eine Achse 55 der in dem zweiten Schenkel 4 gelagerten Facettenschraube 50. Der Divergenzwinkel β beträgt zwischen 15° und 20°, in dem dargestellten Ausführungsbeispiels etwa 18°. Durch diese Abragung nach schräg unten erreicht der Flügelfortsatz 7 eine effektivere Abschirmung des von der Lamina 91 umgrenzten Spinalkanals 96 gegenüber eindringenden Knochenstücken, die insbesondere bei der Resektion des Freiraums 90 angefallen sind. Ein unerwünschtes Eindringen von solchen Knochenstücken hätte die für den Patienten sehr negative Konsequenz, dass es dadurch induziert wiederum zu Druckbelastungen auf den im Spinalkanal 96 laufenden Nervensträngen kommen kann, wodurch der angestrebte Operationserfolg nicht mehr erreicht würde.

Eine weitere Funktionalität des Flügelfortsatzes 7 besteht darin, dass es zusätzlich zur mechanischen Aussteifung dient. Zum einen verleiht es dem Brückenteil 20 eine größere mechanische Stabilität. Der Flügelfortsatz 7 ist einstückig mit dem Brückenteil 20 ausgeführt. Er kann dank der planen Gestaltung seiner Außenseite 70 an der Resektionsfläche 94 bündig anliegen, wobei eine kraftschlüssige Anlage nicht erforderlich ist. Jedoch gilt, dass je geringer der dazwischen befindliche Spalt ist, desto besser ist die Schutzwirkung gegenüber eindringendem Material, insbesondere Knochenstücken wie oben erläutert. Eine möglichst geringe Spaltweite bietet ferner den Vorteil, dass der Flügelfortsatz 7 als Notträger fungieren kann. Sollte sich die Befestigung des Brückenteils 20 an der Verankerung in dem zweiten Schenkel 4 lösen (beispielsweise bei einem Versagen der Scherstoppeinrichtung 5 durch Bruch der Facettenschraube 50), so kann sich dann die Lamina 91 mit ihrer Resektionsfläche 94 nur soweit bewegen, bis sie an der planen Außenseite 70 des Flügelfortsatzes 7 anliegt und nunmehr von dieser getragen ist. Damit ist die Lamina 91 weiterhin unterstützt und ihr Kollabieren wird wirksam verhindert.

In Figur 5a), b) ist eine dritte Ausführungsform dargestellt. Sie unterscheidet sich von der in Figur 4a), b) dargestellten zweiten Ausführungsform im Wesentlichen nur dadurch, dass ein vergrößerter Flügelfortsatz 7' eingesetzt ist. Im Übrigen gelten die zu der zweiten Ausführungsform vorstehend gegebenen Erläuterungen entsprechend.

Das erfindungsgemäße Verstärkungsimplantat 1 ist vorzugsweise Teil eines Implantatsatzes, wie er in Figur 3 dargestellt ist. Man erkennt zeilenweise angeordnet die verschiedenen Typen, die sich in Bezug auf ihre Größe unterscheiden. Für jede Größe ist das Verstärkungsimplantat sowohl in einer Version für die rechtseitige Implantation (rechte Hälfte von Figur 3) wie auch in einer Version zur linksseitigen Implantation (linke Hälfte von Figur 3) vorgesehen. Es gibt jeweils eine Version ohne Flügelfortsatz, eine Version mit kurzem Flügelfortsatz 7 und eine Version mit großem Flügelfortsatz 7'.

## Patentansprüche

1. Verstärkungs-Implantat zum Einsetzen in die Lamina (91) eines Wirbels (9), das einen Grundkörper mit Anlageflächen an den Wirbel und eine Befestigungseinrichtung umfasst,
ein Freitrageteil (2) zur Überspannung eines resezierten Bereichs (92) der Lamina (91) und an gegenüberliegenden Enden des Freitrageteils (2) je ein Verankerungsteil, wobei ein erster Verankerungsteil mit einer Druckfläche (30) zur Anlage am Dornfortsatz (90) des Wirbels (9) und ein zweiter Verankerungsteil mit einer Querschubfläche (40) zur Auflage auf einer Außenseite der Lamina (91) ausgebildet sind, und die Druckfläche (30) und die Querschubfläche (40) einen stumpfen Winkel (α) einschließen, wobei an der Querschubfläche (40) als Befestigungseinrichtung eine Scherstoppeinrichtung (5), mit einer Facettenschraube (50) als Befestigungsstift angeordnet ist, und an die Querschubfläche (40) ein zur Überspannung des resezierten Bereichs (92) der Lamina (91) lasttragender Bereich des Freitrageteils (2) anschließt, wobei der Grundkörper einen ersten Schenkel (3), an dem die Druckfläche (30) angeordnet ist, und einen zweiten Schenkel (4) aufweist, an dem die Querschubfläche (40) angeordnet ist,
**dadurch gekennzeichnet dass**,
in mindestens einem der Schenkel (3, 4) ein Schwenkgelenk (6) für den Befestigungsstift angeordnet ist, das so ausgebildet ist, dass der Befestigungsstift um 10° bis 20° in jeder Richtung um eine Mittellage beweglich ist und in seiner Mittellage um mindestens 10° und höchstens 30° von einer Lotrechten (67) der Querschubfläche (40) abweicht.

2. Verstärkungs-Implantat nach Anspruch 1, **dadurch gekennzeichnet dass**, das Schwenkgelenk (6) einen kalottenförmigen Aufnahmesitz (60) und einen darin gelagerten Ring (61), durch den der Befestigungsstift geführt ist, aufweist.

3. Verstärkungs-Implantat nach Anspruch 2, **dadurch gekennzeichnet dass**, der Ring (61) eine Rotationssperre aufweist, welche ihn drehfest gegenüber dem Aufnahmesitz (60) hält.

4. Verstärkungs-Implantat nach einem der Ansprüche 1 bis 3, **dadurch gekennzeichnet dass**, an dem anderen Schenkel (4,3) ein weiteres Schwenkgelenk (6) mit Befestigungsstift angeordnet ist, und die Schwenkgelenke (6) so ausgebildet sind, dass Befestigungsstifte der beiden Schenkel (3, 4) in Normallage in einer Ebene liegen.

5. Verstärkungs-Implantat nach Anspruch 4, **dadurch gekennzeichnet dass**, einer der Befestigungsstifte in seiner Normallage in der Druckfläche (30) im implantierten Zustand auf ein contralaterales Facettengelenk (95') des Wirbels (9) ausgerichtet ist.

6. Verstärkungs-Implantat nach einem der vorangehenden Ansprüche, **dadurch gekennzeichnet, dass** der Lastkräfte von der Querschubfläche zur Druckfläche übertragende Bereich (20) des Freitrageteils (2) überschneidungsfrei ist in Bezug auf eine durch die Querschubfläche (40) definierte Ebene (24).

7. Verstärkungs-Implantat nach einem der Ansprüche 1 bis 5, **dadurch gekennzeichnet dass**, an der Querschubfläche (4) ein Flügelfortsatz (7) vorgesehen ist, der von der Querschubfläche (40) abragt.

8. Verstärkungs-Implantat nach Anspruch7, **dadurch gekennzeichnet dass**, der Flügelfortsatz (7) mit seinen planen von der Druckfläche (30) wegweisenden Außenseite (70) parallel zur Druckfläche (30) ausgerichtet ist.

9. Verstärkungs-Implantat nach Anspruch 8, **dadurch gekennzeichnet dass**, der Flügelfortsatz (7) eine Verstärkungsrippe (71) an seiner zur Druckfläche (30) weisenden Innenseite aufweist.

10. Verstärkungs-Implantat nach einem der Ansprüche 7 bis9, **dadurch gekennzeichnet dass**, der Flügelfortsatz (7) im Übergangsbereich von der Querschubfläche (40) zum Freitrageteil (2) angeordnet ist.

11. Verstärkungs-Implantat nach einem der Ansprüche 7 bis10, **dadurch gekennzeichnet dass**, der Flügelfortsatz (7) sich über maximal die halbe Weite der Querschubfläche (40) erstreckt.

12. Verstärkungs-Implantat nach einem der Ansprüche 7 bis11, **dadurch gekennzeichnet dass**, ein Unterrand (72) des Flügelfortsatzes (7) divergierend in Bezug auf eine Achse (55) der Scherstoppeinrichtung ausgerichtet ist.

13. Verstärkungs-Implantat nach einem der Ansprüche 5 bis12, **dadurch gekennzeichnet dass**, der Befestigungsstift eine Schraube (50, 50') ist, die lang oder kurz ist, wobei die lange Schraube bis in einen benachbarten unteren Wirbel (9') reicht und die kurze Schraube nicht so weit reicht, sondern im Wirbel (9) endet.

14. Verstärkungs-Implantat nach Anspruch13, **dadurch gekennzeichnet dass**, die lange Schraube an ihrem Schaft kopfseitig einen gewindefreien Bereich (53, 53') und am Ende ein Gewinde (54, 54') aufweist, wobei der gewindefreie Bereich so bemessen ist, dass er bis zum benachbarten unteren Wirbel (9') reicht.

15. Implantat-Satz zum Einsetzen in die Lamina (91) eines Wirbels (9), umfassend mehrere Verstärkungsimplantate in verschiedenen Größen, die je einen Grundkörper mit Anlageflächen an den Wirbel und eine Befestigungseinrichtung umfassen, wobei ein Freitrageteil (2) zur Überspannung eines resezierten Bereichs (92) der Lamina (91) und an gegenüberliegenden Enden des Freitrageteils je ein Verankerungsteil, wobei ein erster Verankerungsteil mit einer Druckfläche (30) zur Anlage am Dornfortsatz (90) des Wirbels (9) und ein zweiter Verankerungsteil mit einer Querschubfläche (40) zur Auflage auf einer Außenseite der Lamina (91) ausgebildet sind, und die Druckfläche (30) und die Querschubfläche (40) einen stumpfen Winkel (α) einschließen, wobei an der Querschubfläche (40) als Befestigungseinrichtung eine Scherstoppeinrichtung (5) mit einer Facettenschraube (50) als Befestigungsstift angeordnet ist, und die Querschubfläche (40) ein zur Überspannung des resezierten Bereichs (92) der Lamina (91) lasttragender Bereich des Freitrageteils (2) anschließt, wobei der Grundkörper einen ersten Schenkel (3), an dem die Druckfläche (30) angeordnet ist, und einen zweiten Schenkel (4) aufweist, an dem die Querschubfläche (40) angeordnet ist, **dadurch gekennzeichnet, dass** in mindestens einem der Schenkel (3, 4) ein Schwenkgelenk (6) für den Befestigungsstift angeordnet ist, das so ausgebildet ist, dass der Befestigungsstift um 10° bis 20° in jeder Richtung um eine Mittellage beweglich ist und in seiner Mittellage um mindestens 10° und höchstens 30° von einer Lotrechten (67) der Querschubfläche (40) abweicht.

16. Implantatsatz nach Anspruch15, **dadurch gekennzeichnet, dass** die Verstärkungsimplantate nach einem der Ansprüche 1 bis 14 ausgeführt sind.

## Claims

1. Reinforcement implant for insertion into the lamina (91) of a vertebra (9), comprising a main body with bearing surfaces on the vertebra and a fastening device, a cantilever part (2) for spanning a resected area (92) of the lamina (91) and also, at opposite ends of the cantilever part (2), in each case an anchoring part, wherein a first anchoring part is designed with a pressure surface (30) for bearing on the spinous process (90) of the vertebra (9), and a second anchoring part is designed with a transverse thrust surface (40) for bearing on an outer face of the lamina (91), and the pressure surface (30) and the transverse thrust surface (40) enclose an obtuse angle (α), wherein, as fastening device, an anti-shear device (5) having a facet screw (50) as fastening pin is arranged on the transverse thrust surface (40), and the transverse thrust surface (40) is adjoined by a load-bearing area of the cantilever part (2) for spanning the resected area (92) of the lamina (91), wherein the main body has a first limb (3), on which the pressure surface (30) is arranged, and a second limb (4), on which the transverse thrust surface (40) is arranged,
**characterized in that**
a pivot joint (6) for the fastening pin is arranged in at least one of the limbs (3, 4), which pivot joint (6) is designed such that the fastening pin is movable through 10° to 20° in each direction about a centre position and, in its centre position, deviates from a perpendicular (67) of the transverse thrust surface (40) by at least 10° and at most 30°.

2. Reinforcement implant according to Claim 1, **characterized in that** the pivot joint (6) has a cup-shaped receiving seat (60) and, mounted in the latter, a ring (61) through which the fastening pin is guided.

3. Reinforcement implant according to Claim 2, **characterized in that** the ring (61) has a rotation barrier, which holds it secure against rotation with respect to the receiving seat (60).

4. Reinforcement implant according to one of Claims 1 to 3, **characterized in that** a further pivot joint (6) having a fastening pin is arranged on the other limb (4, 3), and the pivot joints (6) are designed such that fastening pins of the two limbs (3, 4) lie in one plane in the normal position.

5. Reinforcement implant according to Claim 4, **characterized in that** one of the fastening pins in its normal position in the pressure surface (30) is oriented, in the implanted state, toward a contralateral facet joint (95') of the vertebra (9).

6. Reinforcement implant according to one of the preceding claims, **characterized in that** the area (20) of the cantilever part (2) that transfers loading forces from the transverse thrust surface to the pressure surface does not intersect a plane (24) defined by the transverse thrust surface (40).

7. Reinforcement implant according to one of Claims 1 to 5, **characterized in that** a wing extension (7) is provided on the transverse thrust surface (4), which wing extension (7) protrudes from the transverse thrust surface (40).

8. Reinforcement implant according to Claim 7, **characterized in that** the wing extension (7) is oriented parallel to the pressure surface (30) with its plane outer face (70) which is directed away from the pressure surface (30).

9. Reinforcement implant according to Claim 8, **characterized in that** the wing extension (7) has a reinforcement rib (71) on its inner face directed toward the pressure surface (30).

10. Reinforcement implant according to one of Claims 7 to 9, **characterized in that** the wing extension (7) is arranged in the transition area from the transverse thrust surface (40) to the cantilever part (2).

11. Reinforcement implant according to one of Claims 7 to 10, **characterized in that** the wing extension (7) extends over at most half the width of the transverse thrust surface (40) .

12. Reinforcement implant according to one of Claims 7 to 11, **characterized in that** a lower edge (72) of the wing extension (7) has a diverging orientation with respect to an axis (55) of the anti-shear device.

13. Reinforcement implant according to one of Claims 5 to 12, **characterized in that** the fastening pin is a screw (50, 50') that is long or short, wherein the long screw reaches into an adjacent lower vertebra (9'), and the short screw does not reach as far and instead ends within the vertebra (9).

14. Reinforcement implant according to Claim 13, **characterized in that** the shaft of the long screw has a threadless area (53, 53') toward its head and a thread (54, 54') at the end, the threadless area being dimensioned such that it reaches as far as the adjacent lower vertebra (9').

15. Implant set for insertion into the lamina (91) of a vertebra (9), comprising a plurality of reinforcement implants of various sizes, each comprising a main body with bearing surfaces on the vertebra and a fastening device, wherein
a cantilever part (2) for spanning a resected area (92) of the lamina (91) and also, at opposite ends of the cantilever part, in each case an anchoring part, wherein a first anchoring part is designed with a pressure surface (30) for bearing on the spinous process (90) of the vertebra (9), and a second anchoring part is designed with a transverse thrust surface (40) for bearing on an outer face of the lamina (91), and the pressure surface (30) and the transverse thrust surface (40) enclose an obtuse angle (α), wherein, as fastening device, an anti-shear device (5) having a facet screw (50) as fastening pin is arranged on the transverse thrust surface (40), and the transverse thrust surface (40) is adjoined by a load-bearing area of the cantilever part (2) for spanning the resected area (92) of the lamina (91), the main body has a first limb (3), on which the pressure surface (30) is arranged, and a second limb (4), on which the transverse thrust surface (40) is arranged, **characterized in that** a pivot joint (6) for the fastening pin is arranged in at least one of the limbs (3, 4), which pivot joint (6) is designed such that the fastening pin is movable through 10° to 20° in each direction about a centre position and, in its centre position, deviates from a perpendicular (67) of the transverse thrust surface (40) by at least 10° and at most 30°.

16. Implant set according to Claim 15, **characterized in that** the reinforcement implants are designed according to one of Claims 1 to 14.

## Revendications

1. Implant de renforcement destiné à être inséré dans la lame vertébrale (91) d'une vertèbre (9), lequel comporte un corps de base doté de surfaces d'appui contre la vertèbre et un dispositif de fixation,
une partie en porte-à-faux (2) servant à enjamber une région réséquée (92) de la lame vertébrale (91) et, aux extrémités opposées de la partie en porte-à-faux (2), respectivement une partie d'ancrage, dans lequel une première partie d'ancrage est réalisée de manière à présenter une surface de pression (30) pour l'appui contre l'apophyse épineuse (90) de la vertèbre (9) et une deuxième partie d'ancrage est réalisée de manière à présenter une surface de poussée transversale (40) destinée à reposer sur un côté extérieur de la lame vertébrale (91), et la surface de pression (30) et la surface de poussée transversale (40) forment un angle obtus (α), dans lequel un dispositif anti-cisaillement (5), doté d'une vis pour facette (50) en tant que goupille de fixation, est disposé sur la surface de poussée transversale (40) en tant que dispositif de fixation, et une région de support de charge de la partie en porte-à-faux (2) se raccorde à la surface de poussée transversale (40), laquelle région de support de charge est destinée à enjamber la région réséquée (92) de la lame vertébrale (91), dans lequel le corps de base comprend une première branche (3) sur laquelle la surface de pression (30) est disposée et une deuxième branche (4) sur laquelle la surface de poussée transversale (40) est disposée,
**caractérisé en ce**
**qu'**une articulation pivotante (6) pour la goupille de fixation est disposée dans au moins l'une des branches (3, 4), laquelle articulation pivotante est réalisée de telle sorte que la goupille de fixation soit mobile sur 10° à 20° dans chaque sens autour d'une position centrale et, dans sa position centrale, s'écarte d'au moins 10° et d'au plus 30° d'une normale (67) à la surface de poussée transversale (40).

2. Implant de renforcement selon la revendication 1, **caractérisé en ce que** l'articulation pivotante (6) comprend un logement (60) en forme de calotte et une bague (61) montée dans celui-ci, bague à travers laquelle la goupille de fixation est guidée.

3. Implant de renforcement selon la revendication 2, **caractérisé en ce que** la bague (61) comprend un blocage anti-rotation, lequel maintient celle-ci de manière bloquée en rotation par rapport au logement (60).

4. Implant de renforcement selon l'une des revendications 1 à 3, **caractérisé en ce qu'**une autre articulation pivotante (6) dotée d'une goupille de fixation est disposée sur l'autre branche (4, 3), et les articulations pivotantes (6) sont réalisées de telle sorte que les goupilles de fixation des deux branches (3, 4) se situent dans un plan dans la position normale.

5. Implant de renforcement selon la revendication 4, **caractérisé en ce que** l'une des goupilles de fixation est, dans sa position normale dans la surface de pression (30) à l'état implanté, orientée vers une articulation de facette (95') contralatérale de la vertèbre (9).

6. Implant de renforcement selon l'une des revendications précédentes, **caractérisé en ce que** la région (20) de la partie en porte-à-faux (2) transmettant des forces de charge de la surface de poussée transversale à la surface de pression ne croise pas un plan (24) défini par la surface de poussée transversale (40).

7. Implant de renforcement selon l'une des revendications 1 à 5, **caractérisé en ce qu'**un prolongement formant ailette (7) est prévu sur la surface de poussée transversale (4), lequel fait saillie à partir de la surface de poussée transversale (40).

8. Implant de renforcement selon la revendication 7, **caractérisé en ce que** le prolongement formant ailette (7) est orienté parallèlement à la surface de pression (30) par son côté extérieur (70) plan opposé à la surface de pression (30).

9. Implant de renforcement selon la revendication 8, **caractérisé en ce que** le prolongement formant ailette (7) comprend une nervure de renforcement (71) sur son côté intérieur tourné vers la surface de pression (30).

10. Implant de renforcement selon l'une des revendications 7 à 9, **caractérisé en ce que** le prolongement formant ailette (7) est disposé dans la région de transition de la surface de poussée transversale (40) à la partie en porte-à-faux (2).

11. Implant de renforcement selon l'une des revendications 7 à 10, **caractérisé en ce que** le prolongement formant ailette (7) s'étend sur au maximum la moitié de l'étendue de la surface de poussée transversale (40).

12. Implant de renforcement selon l'une des revendications 7 à 11, **caractérisé en ce qu'**un bord inférieur (72) du prolongement formant ailette (7) est orienté de manière divergente par rapport à un axe (55) du dispositif anti-cisaillement.

13. Implant de renforcement selon l'une des revendications 5 à 12, **caractérisé en ce que** la goupille de fixation est une vis (50, 50') qui est longue ou courte, la vis longue s'étendant jusque dans une vertèbre inférieure (9') adjacente et la vis courte ne s'étendant pas aussi loin, mais se terminant dans la vertèbre (9).

14. Implant de renforcement selon la revendication 13, **caractérisé en ce que** la vis longue comprend, sur sa tige, côté tête, une région (53, 53') dépourvue de filetage et, à l'extrémité, un filetage (54, 54'), la région dépourvue de filetage étant dimensionnée de telle sorte qu'elle s'étend jusqu'à la vertèbre inférieure (9') adjacente.

15. Jeu d'implants destiné à être inséré dans la lame vertébrale (91) d'une vertèbre (9), comportant plusieurs implants de renforcement de différentes tailles qui comportent respectivement un corps de base doté de surfaces d'appui contre la vertèbre et un dispositif de fixation, dans lequel une partie en porte-à-faux (2) servant à enjamber une région réséquée (92) de la lame vertébrale (91) et, aux extrémités opposées de la partie en porte-à-faux, respectivement une partie d'ancrage, dans lequel une première partie d'ancrage est réalisée de manière à présenter une surface de pression (30) pour l'appui contre l'apophyse épineuse (90) de la vertèbre (9) et une deuxième partie d'ancrage est réalisée de manière à présenter une surface de poussée transversale (40) destinée à reposer sur un côté extérieur de la lame vertébrale (91), et la surface de pression (30) et la surface de poussée transversale (40) forment un angle obtus (α), dans lequel un dispositif anti-cisaillement (5), doté d'une vis pour facette (50) en tant que goupille de fixation, est disposé sur la surface de poussée transversale (40) en tant que dispositif de fixation, et une région de support de charge de la partie en porte-à-faux (2) se raccorde à la surface de poussée transversale (40), laquelle région de support de charge est destinée à enjamber la région réséquée (92) de la lame vertébrale (91), dans lequel le corps de base comprend une première branche (3) sur laquelle la surface de pression (30) est disposée et une deuxième branche (4) sur laquelle la surface de poussée transversale (40) est disposée, **caractérisé en ce qu'**une articulation pivotante (6) pour la goupille de fixation est disposée dans au moins l'une des branches (3, 4), laquelle articulation pivotante est réalisée de telle sorte que la goupille de fixation soit mobile sur 10° à 20° dans chaque sens autour d'une position centrale et, dans sa position centrale, s'écarte d'au moins 10° et d'au plus 30° d'une normale (67) à la surface de poussée transversale (40).

16. Jeu d'implants selon la revendication 15, **caractérisé en ce que** les implants de renforcement sont réalisés selon l'une des revendications 1 à 14.
